# EUROPEAN PATENT SPECIFICATION

(11) **EP 1 149 892 B1**
(45) Date of publication and mention of the grant of the patent: **04.04.2007**
(21) Application number: 00978068.5
(22) Date of filing: 01.12.2000
(51) Int. Cl.: C11D 1/94, C11D 1/86, C11D 1/46

(54) **SURFACTANT COMPOSITION**
OBERFLAECHENAKTIVE ZUSAMMENSETZUNG
COMPOSITION DE TENSIOACTIF

(30) Priority: 01.12.1999 JP 34164999
(43) Date of publication of application: 31.10.2001
(73) Proprietor: KAO CORPORATION, Tokyo 103-8210 (JP)
(72) Inventor: SAKAI, Takaya, Kao Corporation, Wakayama-shi, Wakayama 640-8580 (JP); TETSU, Makio, Kao Corporation, Wakayama-shi, Wakayama 640-8580 (JP); KUBO, Makoto, Kao Corporation, Wakayama-shi, Wakayama 640-8580 (JP); FUJIU, Akira, Kao Corporation, Wakayama-shi, Wakayama 640-8580 (JP)
(74) Representative: HOFFMANN EITLE
(86) International application number: PCT/JP2000/008539
(87) International publication number: WO 2001/040421

(56) References cited:
- JP-A- 7 194 958
- JP-A- 9 137 190
- JP-A- 10 330 783

## Description

### Technical Field of the Invention

The present invention relates to a surfactant composition having an excellent foamability, thickening property, water solubility and stability.

### Prior Art

Not only a high detergency but also an abundant foaming are requested to a detergent such as a shampoo, a rinse, a solid soap, a body shampoo, a detergent for kitchens, a detergent for clothing and a detergent for dwellings. One which has been widely used as the main base in many products is an anionic surfactant such as an alkyl benzenesulfonate, an alkyl sulfate salt and a polyoxyethylene alkyl sulfate salt. However, although any thereof usually shows its excellent detergency and foaming, there is a problem therein that, when solids, a stain derived from silicone or the like is present, the detergency and foamability significantly lower.

In order to improve such a lowering in detergency and/or foamability, various co-surfactants besides the main base have been investigated. There have been first proposed a fatty acid diethanolamide which has been actually and widely used in the products and then proposed in recent years a fatty acid monoethanolamide (WO 98/00507, WO 97/44434, JP-A 11-80785, etc.), a nonionic surfactant based on a sugar amide (WO 94/12610), an acylsarcosinate (WO 96/06596), etc.

However, although these detergent compositions are improved in detergency and foamability, they are not well satisfactory yet. Moreover, since the detergents described above directly contact with the skin daily, they are demanded to exhibit a high performance at a weakly acidic pH region which is mild to the skin. However, in the conventional surfactant system, there has been mentioned such a problem that, even when the foaming is good in from neutral to basic regions, foaming does not take place at all in an acidic region.

### Disclosure of the Invention

A purpose of the present invention is to provide a surfactant composition having an excellent foamability, thickening property, water solubility and stability even within a broad pH range, even in the presence of soil.

The present invention provides a surfactant composition comprising the below shown component (a) and component (b) and a surfactant other than (a) and (b) at the weight ratio of (a)/(b) of from 99.99/0.01 to 80/20 or from 99.9/0.1 to 70/30.
(a) a compound (I) represented by the following formula (I) : 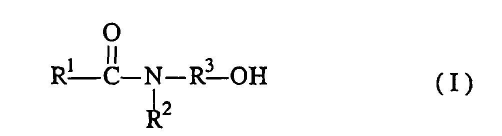 in the formula (I), R¹CO- is a saturated or unsaturated acyl group which has 6 to 24 carbon atoms and which may have a hydroxy group; R² is a linear or branched alkyl group having 1 to 3 carbon atoms; and R³ is a linear or branched alkylene group having 1 to 6 carbon atoms or alkenylene group having 2 to 6 carbon atoms.
(b) at least one compound selected from the group consisting of the compounds (II) represented by the formula (II) and the compounds (III) represented by the formula (III): 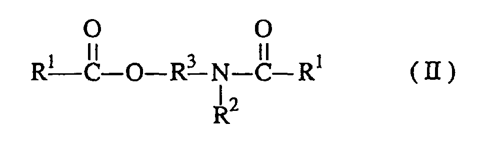 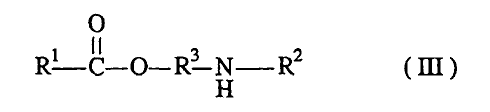 in the formulae (II) and (III), R¹CO-, R² and R³ have the same meanings as above and R¹, R² and R³ are either the same as or different from one another in each compound.

### Embodiments of worked invention

In the component (a) of the present invention, R¹CO- is an acyl group as mentioned above. It is preferably a saturated or unsaturated acyl group having 8 to 18 carbon atoms. It includes for example an acyl group derived from octanoic acid, decanoic acid, dodecanoic acid, tetradecanoic acid, hexadecanoic acid, octadecanoic acid, docosanoic acid, linoleic acid, 2-ethylhexanoic acid, 2-octylundecanoic acid, isostearic acid, oleic acid, coco-fatty acid, a palm oil-fatty acid, a palm kernel oil-fatty acid, a beef tallow-fatty acid, etc. The more preferable is an acyl group derived from octanoic acid, decanoic acid, dodecanoic acid, tetradecanoic acid, hexadecanoic acid, octadecanoic acid, oleic acid, coco-fatty acid, palm oil-fatty acid, palm kernel oil-fatty acid and beef tallow-fatty acid. Among these acyl groups, that containing not less than 50 % by weight of acyl groups derived from fatty acids having 12 to 14 carbon atoms is preferable. More preferable is that containing 40 to less than 100 percent by weight of acyl groups derived from fatty acids having 12 carbon atoms.

In order not to lower foamability, R² is a linear or branched alkyl group having 1 to 3 carbon atoms, preferably methyl group or ethyl group. Methyl group is in particular preferable. In order not to lower the surface-active performance, R³ is a linear or branched alkylene having 1 to 6 carbon atoms or an alkenylene group having 2 to 6 carbon atoms. It is preferably a linear or branched alkylene group having 2 or 3 carbon atoms.

The component (a) includes, for instance, N-(2-hydroxyethyl)-N-methyl octanamide, N-(2-hydroxyethyl)-N-methyl decanamide, N-(2-hydroxyethyl)-N-methyl dodecanamide, N-(2-hydroxyethyl)-N-methyl tertadecanamide, N-(2-hydroxyethyl)-N-methyl hexadecanamide, N-(2-hydroxyethyl)-N-methyl octadecanamide, N-(2-hydroxyethyl)-N-methyl-coco-fatty carboxamide, N-(2-hydroxyethyl)-N-methyl-palm kernel oil-fatty carboxamide, N-(2-hydroxypropyl)-N-ethyl dodecanamide, N-ethyl-N-(2-hydroxypropyl) oleamide and N-ethyl-N-(2-hydroxypropyl) isostearamide.

A method for producing the component (a) is not particularly limited. For example, it can be produced by a condensation reaction or a de-alcohol reaction of a fatty acid or a lower alcohol ester of a fatty acid with an alkanolamine, a reaction of a fatty acid halide with an alkanolamine in the presence of an alkaline catalyst, an ester-amide exchanging reaction (an aminolysis) between fats and oils and an alkanolamine or others. The product obtained by such a method may often contain small amounts of fatty acids, inorganic salts, glycerol and the like, but they does not at all affect properties of the product.

The component (b) is selected from the compounds represented by the above-mentioned formulae (II) and (III), respectively. It is not necessarily single, but plural species may be mixed.

R¹CO-, R² and R³ of the formulae (II) and (III) have the same meanings as those of the formula (I) . In each compound, R¹, R² and R³ may be the same as or different from one another.

The compound represented by the formula (II) includes, for instance, N-octanoyl-N-methylaminoethyl octanoate, N-octanoyl-N-methylaminoethyl decanoate, N-octanoyl-N-methylaminoethyl dodecanoate, N-octanoyl-N-methylaminoethyl tetradecanoate, N-octanoyl-N-methylaminoethyl hexadecanoate, N-octanoyl-N-methylaminoethyl octadecanoate, N-dodecanoyl-N-methylaminoethyl octanoate, N-dodecanoyl-N-methylaminoethyl decanoate, N-dodecanoyl-N-methylaminoethyl dodecanoate, N-dodecanoyl-N-methylaminoethyl tetradecanoate, N-dodecanoyl-N-methylaminoethyl hexadecanoate, N-dodecanoyl-N-methylaminoethyl octadecanoate, N-dodecanoyl-N-ethylaminohexyl octanoate, N-dodecanoyl-N-ethylaminohexyl decanoate, N-dodecanoyl-N-ethylaminohexyl dodecanoate, N-dodecanoyl-N-ethylaminohexyl tetradecanoate, N-dodecanoyl-N-ethylaminohexyl hexadecanoate, N-dodecanoyl-N-ethylaminohexyl octadecanoate, coco-fatty acid (N-cocoyl-N-methylaminoethyl)ester and palm kernel oil fatty acid (N-palm kerneloyl-N-methylaminoethyl) ester.

Examples of the compound represented by the formula (III), for instance, include 2- (methylamino) ethyl octanoate, 2-(methylamino)ethyl decanoate, 2-(methylamino)-ethyl dodecanoate, 2-(methylamino)ethyl tetradecanoate, 2-(methylamino)ethyl hexadecanoate, 2-(methylamino)ethyl octadecanoate, 2-(methylamino)ethyl oleate, 2-(ethylamino)ethyl octanoate, 2-(ethylamino)ethyl dodecanoate, 2-(ethylamino)ethyl tetradecanoate, 2-(ethylamino)ethyl hexadecanoate, 2-(ethylamino)ethyl octadecanoate, 2-(ethylamino)ethyl oleate, 2-(methylamino)propyl octanoate, 2-(methylamino)propyl decanoate, 2- (methylamino)propyl dodecanoate, 2-(methylamino)propyl tetradecanoate, 2-(methylamino)propyl hexadecanoate, 2- (methylamino)propyl octadecanoate, 2-(methylamino)propyl oleate, a coco-fatty acid(2-ethylaminoethyl) ester and palm kernel fatty acid (2-ethylaminoethyl) ester.

Further, the blending ratio of the component (a) to the component (b), the weight ratio of (a) / (b), is from 99.99/0.01 to 80/20 or 99.9/0.1 to 70/30, preferably from 98/2 to 90/10 in view of foamability, thickening and stability.

With regard to the blending proportions of the compounds (I), (II) and (III) of the composition of the present invention to obtain a good surface activity, the compound (I) is preferably 80 to 99.99 % by weight to the sum total of them, more preferably 90 to 98 % by weight. The compound (II) is preferably 0.01 to 20 % by weight, more preferably 2 to 10 % by weight. The compound (III) is preferably 0 to 5 % by weight.

A very high foamability and thickening property can be exhibited in the present invention by combining another surfactant (hereinafter, referred to as "other surfactant") than (a) and (b) with (a) and (b). The total amount of the components (a) and (b) of the composition of the present invention can be optionally selected, depending upon characters of the other surfactant. In view of water solubility, stability, foamability and thickening property, the blending ratio of the components (a) and (b) in total to the other surfactant is preferably from 0.5/99.5 to 50/50 by weight.

The other surfactant used in the invention may be at least one selected from a sulfate-based or sulfonate-based surfactant such as an alkyl sulfate, a polyoxyethylene alkyl sulfate and an alkylbenzene sulfonate, an amine oxide such as an alkyldimethyl amine oxide and amidopropyldimethyl amine oxide and an amide group-containing betaine such as amidopropyl dimethylcarbobetaine. One or two or more can be used among them. It is preferable that one or more sulfate-based surfactants are co-present.

The surfactant composition of the present invention can be improved in viscosity by further containing glycerol. The amount of glycerol to the sum total of the components (a) and (b) and the amount of the surfactant other than the components (a) and (b) is preferably not more than 20 % by weight, more preferably from 0.01 to 15 % by weight. Alternatively the blending ratio of the sum total of the component (a), the component (b) and glycerol to the other surfactant is preferably from 0.5/99.5 to 50/50, more preferably from 1/99 to 25/75 by weight. Glycerol may be added separately. It may contain that produced in the syntheses of the component (a).

The surfactant composition of the present invention can exhibit desired performances such as foamability and thickening property at a pH of 4.0 to 11.0. It is useful as the base for a detergent.

### Examples

Examples 1 to 7 and Comparative Examples 1 to 5

Using the components as shown below, surfactant compositions having the formulations as shown in Table 1 were prepared and tested in foamability in the below mentioned method. Results are shown in Table 1.

### <Components>

### Component (a)

Compound 1: N- (2-hydroxyethyl)-N-methyl lauramide
Compound 2: N- (2-hydroxyethyl)-N-methyl coco-fatty carboxamide
Compound 3: N-(2-hydroxyethyl)-N-methyl palm kernel oil-fatty carboxamide

### Component (b)

Compound 4: N-Lauroyl-N-methylaminoethyl laurate
Compound 5: (N-cocoyl-N-methylaminoethyl) coco-fatty carboxylate
Compound 6: (N-palm kerneloyl-N-methylaminoethyl) palm kernel-fatty carboxylate
Compound 7: 2- (methylamino) ethyl laurate
Compound 8: (2-ethylaminoethyl) coco-fatty carboxylate
Compound 9: (2-ethylaminoethyl) palm kernel-fatty carboxylate

### Other Surfactants

- AES:: sodium polyoxyethylene(3) laurylsulfate
- LAPB:: lauroylamidopropyl dimethylcarbobetain
- LDMAO:: long-chain alkyl(C₁₂) dimethylamine oxide
- MEA:: lauroyl mono-ethanolamide
- DEA:: lauroyl di-ethanolamide
- EAA:: polyoxyethylene(2)coco-fatty monoethanolamide

### <Method for evaluating the foamability>

30 mL of an aqueous solution of the surfactant composition, diluted in deionized water to 20-fold was prepared and adjusted with citric acid to have a given pH value. The aqueous solution was placed in a 300-mL mess cylinder having the inner diameter of 3 cm, equipped with a stop cock and shaken at 25°C for 10 seconds with an amplitude of 10 cm 20 times. The volume (mL) of foam was immediately determined.

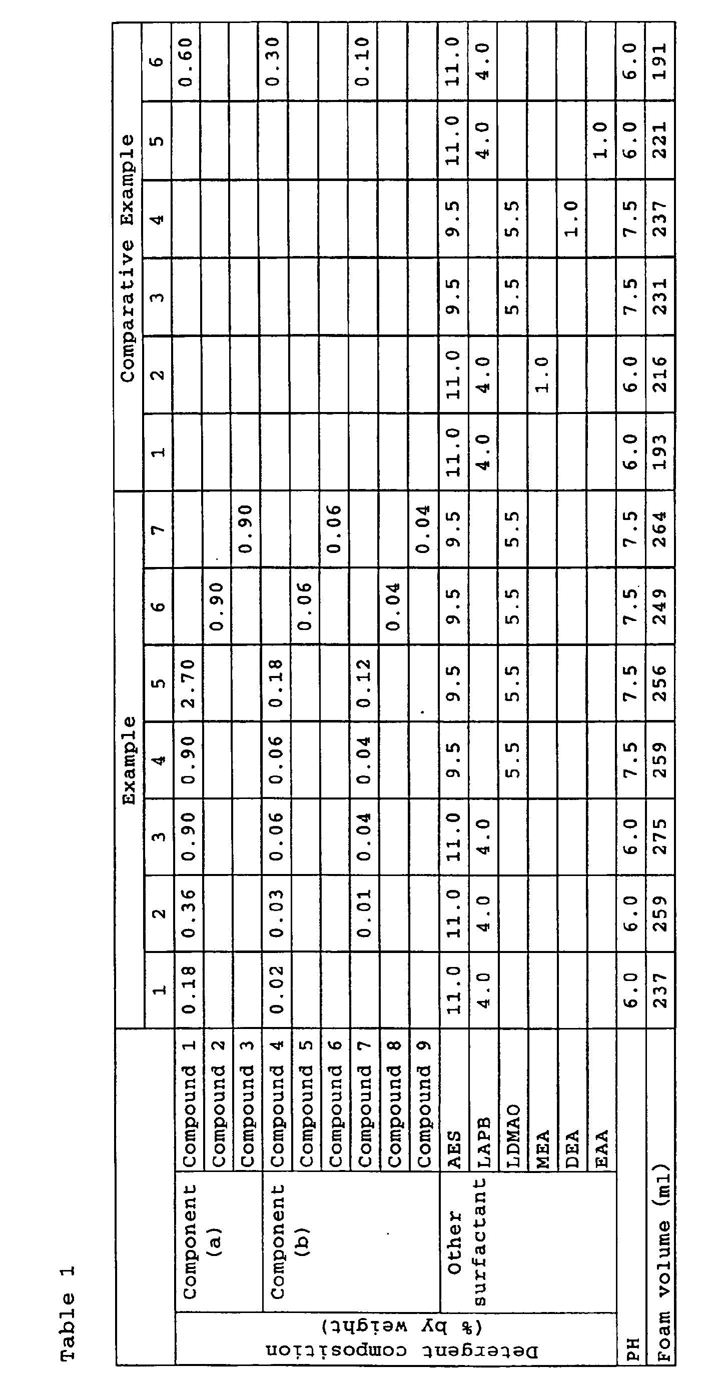

### Example 8 to 16 and Comparative Example 8 to 11

Using components shown in Table 2, surfactant compositions having formulations as shown in Table 2 were prepared and tested in foamability in the same way as Example 1. Results are shown in Table 2.

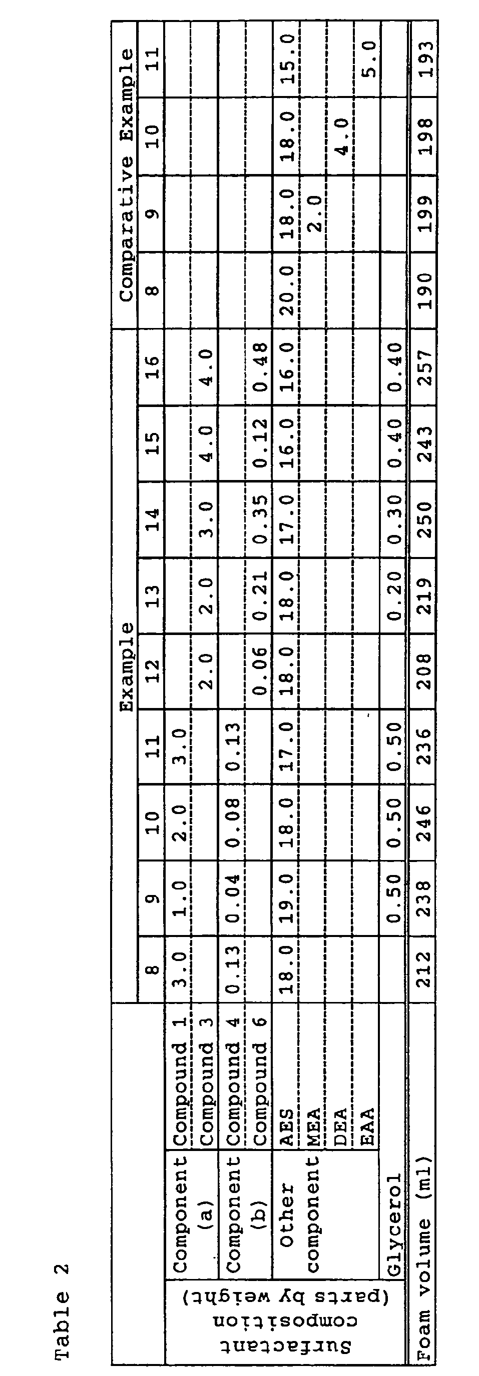

### Examples 17 to 20 and Comparative Examples 12 to 16

Using components shown in Table 3, surfactant compositions having formulations as shown in Table 3 were prepared. The viscosity thereof was determined at 25 °C with an ordinary Brookfield viscometer. Results are shown in Table 3.

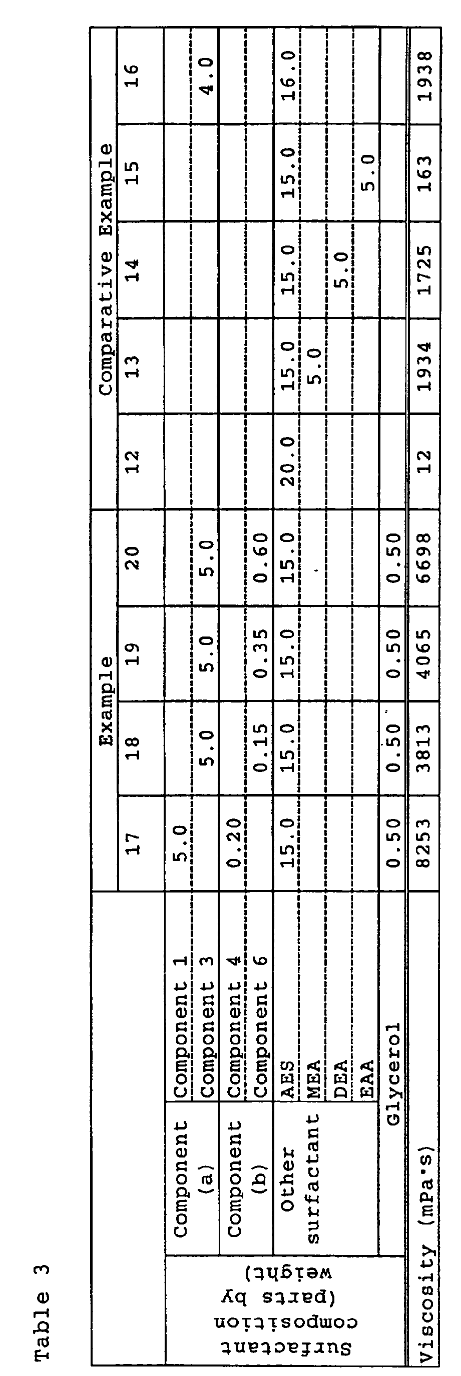

## Claims

1. A surfactant composition containing the below shown component (a), the below shown component (b) and an other surfactant than (a) and (b) at the weight ratio of (a)/(b) of from 99.99/0.01 to 80/20:
(a) the compound (hereinafter called the compound (I)) represented by the formula (I): 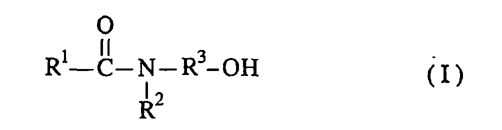 wherein R¹CO- is a saturated or unsaturated acyl group which has 6 to 24 carbon atoms and may have a hydroxy group; R² is a linear or branched alkyl group having 1 to 3 carbon atoms; and R³ is a linear or branched alkylene group having 1 to 6 carbon atoms or an alkenylene group having 2 to 6 carbon atoms;
(b) at least one compound selected from the compound (hereinafter called the compound (II)) represented by the formula (II) and the compound (hereinafter called the compound (III)) represented by the formula (III): 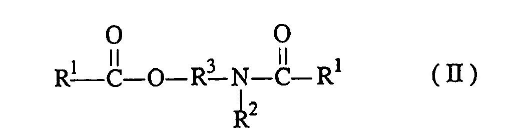 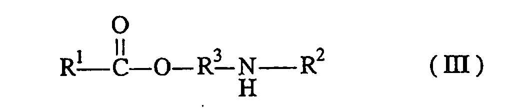
wherein R¹CO-, R² and R³ have the same meanings as above and R¹, R² and R³ may be the same as or different from one another.

2. The composition as claimed in Claim 1, wherein the blending proportions of the compounds (I), (II) and (III) to the sum total of them are 80 to 99.99 % by weight of the compound (I), 0.01 to 20 % by weight of the compound (II) and 0 to 5 % by weight of the compound (III).

3. The composition as claimed in Claim 2, wherein the blending ratio of the sum total of the components (a) and (b) to the other surfactant than the components (a) and (b) is from 0.5/99.5 to 50/50 by weight.

4. The composition as claimed in any of Claim 1 to 3, which comprises glycerol in an amount of 20 % by weight or less to the sum total of the components (a) and (b) and the other surfactant than the components (a) and (b).

5. The composition as claimed in Claim 4, in which the blending ratio of the sum total of the components (a) and (b) and glycerol to the other surfactant than the components (a) and (b) is from 0.5/99.5 to 50/50 by weight.

## Patentansprüche

1. Tensidzusammensetzung, umfassend die unten gezeigte Komponente (a), die unten gezeigte Komponente (b) und ein anderes Tensid als (a) und (b) bei einem Gewichtsverhältnis von (a)/(b) von 99,99/0,01 bis 80/20:
(a) Verbindung mit der Formel (I) (nachfolgend Verbindung (I)): 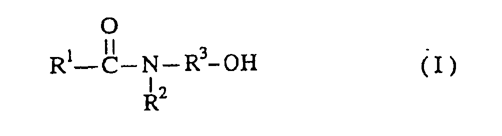 worin R¹CO- eine gesättigte oder ungesättigte Acylgruppe ist, die 6 bis 24 Kohlenstoffatome aufweist und eine Hydroxygruppe haben kann, R² eine lineare oder verzweigte Alkylgruppe mit 1 bis 3 Kohlenstoffatomen ist; und R³ eine lineare oder verzweigte Alkylengruppe mit 1 bis 6 Kohlenstoffatomen oder eine Alkenylengruppe mit 2 bis 6 Kohlenstoffatomen ist;
b) zumindest eine Verbindung, ausgewählt aus der Verbindung mit der Formel (II) (nachfolgend Verbindung (II)) und der Verbindung mit der Formel (III) (nachfolgend Verbindung (III)): 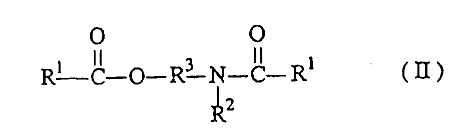 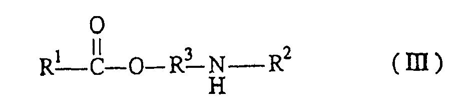
worin R¹CO-, R² und R³ die gleichen Bedeutungen wie oben aufweisen und R¹, R² und R³ gleich oder verschieden voneinander sein können.

2. Zusammensetzung nach Anspruch 1, worin die Mischungsverhältnisse der Verbindungen (I), (II) und (III) zur Gesamtsumme von diesen 80 bis 99,99 Gew.% der Verbindung (I), 0,01 bis 20 Gew.% der Verbindung (II) und 0 bis 5 Gew.% der Verbindung (III) sind.

3. Zusammensetzung nach Anspruch 2, worin das Mischungsverhältnis der Gesamtsumme der Komponenten (a) und (b) zu dem anderen Tensid als den Komponenten (a) und (b) von 0,5/99,5 bis 50/50 ist, bezogen auf das Gewicht.

4. Zusammensetzung nach einem der Ansprüche 1 bis 3, umfassend Glyzerin in einer Menge von 20 Gew.% oder weniger zur Gesamtsumme der Komponenten (a) und (b) und dem anderen Tensid als den Komponenten (a) und (b).

5. Zusammensetzung nach Anspruch 4, worin das Mischungsverhältnis der Gesamtsumme der Komponenten (a) und (b) und Glyzerin zu dem anderen Tensid als den Komponenten (a) und (b) von 0,5/99,5 bis 50/50 ist, bezogen auf das Gewicht.

## Revendications

1. Composition de tensioactif contenant le composant (a) montré ci-dessous, le composant (b) montré ci-dessous et un autre tensioactif que (a) et (b) à un rapport pondéral de (a)/(b) de 99, 99/0,01 à 80/20 :
(a) le composé (ci-après appelé le composé (I)) représenté par la formule (I) : 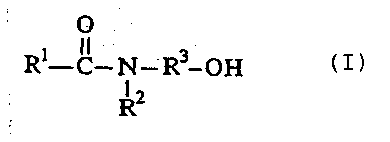 dans laquelle R¹CO- est un groupe acyle saturé ou insaturé qui a 6 à 24 atomes de carbone et peut avoir un groupe hydroxy ; R² est un groupe alkyle linéaire ou ramifié ayant 1 à 3 atomes de carbone; et R³ est un groupe alkylène linéaire ou ramifié ayant 1 à 6 atomes de carbone ou un groupe alcénylène ayant 2 à 6 atomes de carbone ;
(b) au moins un composé choisi à partir du composé (ci-après appelé le composé (II)) représenté par la formule (II) et du composé (ci-après appelé le composé (III)) représenté par la formule (III) 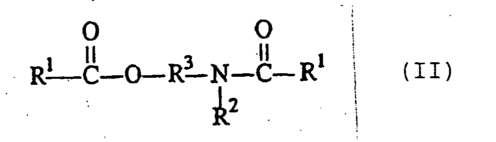 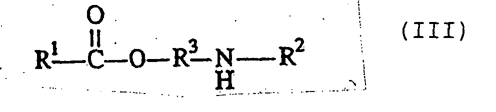
dans lesquelles R¹CO-, R² et R³ ont les mêmes significations que ci-dessus et R¹, R² et R³ peuvent être identiques ou différents les uns des autres.

2. Composition selon la revendication 1, dans laquelle les proportions de mélange des composés (I), (II) et (III) par rapport à la somme totale de ceux-ci sont de 80 à 99,99 % en poids du composé (I), de 0,01 à 20 % en poids du composé (II) et de 0 à 5 % en poids du composé (III).

3. Composition selon la revendication 2, dans laquelle le rapport de mélange de la somme totale des composants (a) et (b) par rapport à l'autre tensioactif que les composants (a) et (b) est de 0,5/99,5 à 50/50 en poids.

4. Composition selon l'une quelconque des revendications 1 à 3, qui comprend du glycérol en une quantité de 20 % en poids ou moins par rapport à la somme totale des composants (a) et (b) et de l'autre tensioactif que les composants (a) et (b).

5. Composition selon la revendication 4, dans laquelle le rapport de mélange de la somme totale des composants (a) et (b) et du glycérol par rapport à l'autre tensioactif que les composants (a) et (b) est de 0,5/99,5 à 50/50 en poids.
